# EUROPÄISCHE PATENTSCHRIFT

(11) **EP 1 948 194 B1**
(45) Veröffentlichungstag und Bekanntmachung des Hinweises auf die Patenterteilung: **02.07.2014**
(21) Anmeldenummer: 06818441.5
(22) Anmeldetag: 09.11.2006
(51) Int. Cl.: A61K 31/663, A61P 19/00

(54) **VERWENDUNG VON CLODRONSÄURE ZUR BEHANDLUNG VON PODOTROCHLOSE**
USE OF CLODRONIC ACID FOR THE TREATMENT OF NAVICULAR SYNDROME
UTILISATION D'ACIDE CLODRONIQUE POUR TRAITER LA PODOTROCHLOSE

(30) Priorität: 09.11.2005 DE 102005053512
(43) Veröffentlichungstag der Anmeldung: 30.07.2008
(73) Patentinhaber: Omnimedic Gbr, D-44141 Dortmund (DE)
(72) Erfinder: FREVEL, Michael, 50181 Bedburg (DE)
(74) Vertreter: Merkle, Gebhard
(86) Internationale Anmeldenummer: PCT/EP2006/010753
(87) Internationale Veröffentlichungsnummer: WO 2007/054309

(56) Entgegenhaltungen:
- WO-A-97/12619
- US-A1- 2001 006 960
- DENOIX J -M ET AL: "Tiludronate as a new therapeutic agent in the treatment of navicular disease: A double-blind placebo-controlled clinical trial." EQUINE VETERINARY JOURNAL, Bd. 35, Nr. 4, Juni 2003 (2003-06), Seiten 407-413, XP009092632 ISSN: 0425-1644
- MCGUIGAN M P ET AL: "A double-blind placebo-controlled trial of biphosphonate in the treatment ofnavicular syndrome" PROCEEDINGS OF THE BRITISH EQUINE VETERINARY ASSOCIATION CONGRESS, X, XX, Bd. 39, 2000, Seite 207, XP001536375
- GRAY A W ET AL: "Generation and activity of equine osteoclasts in vitro: Effects of the bisphosphonate pamidronate (APD)" RESEARCH IN VETERINARY SCIENCE, Bd. 72, Nr. 2, April 2002 (2002-04), Seiten 105-113, XP002460164 ISSN: 0034-5288

## Beschreibung

### GEBIET DER ERFINDUNG

Die vorliegende Erfindung betrifft die Verwendung von Clodronsäure, deren Additionssalze oder Ester, Hydrate oder Salze von Hydraten zur Behandlung von Podotrochlose mit osteoporotischer Beteiligung bei Pferden bzw. die Verwendung von Clodronsäure, deren Additionssalze oder Ester als Wirkstoff zur Herstellung eines Arzneimittels zur Behandlung von Podotrochlose mit osteoporotischer Beteiligung bei Pferden.

### HINTERGRUND DER ERFINDUNG

In der Pferdemedizin ist die Erkrankung "Strahlbeinlahmheit" oder "Podotrochlose", im Volksmund auch "Hufrollenentzündung" genannt, eine weit verbreitete Erkrankung im distalen Abschnitt des Pferdevorderbeins. Die klassische Form der Erkrankung ist als chronische, progressive und degenerative Erkrankung mit Beteiligung des Strahlbeines, des Hufrollenschleimbeutels und der Beugesehne beschrieben worden.

Durch genetische Prädisposition, mangelhafte Aufzucht, schlechtes oder unregelmäßiges Training, starke Belastung und altersbedingt kommt es bei Pferden häufig zu einer Atrophie des Strahlbeines, wodurch eine Demineralisation des Knochens einsetzt. Der subchondrale Knochen wird dann durch die unphysiologischen Druckverhältnisse, welche auf den Knochen wirken, osteoporotisch verändert.

In den Frühstadien der Erkrankung enthält der subchondrale Knochen zahlreiche erweiterte und mit Granulationsgewebe gefüllte Gefäßkanäle, die von Osteoklasten und Osteoblasten umgeben sind. Im Laufe der Pathogenese entwickeln sich osteoklastische Veränderungen, die sich der Kompakta der Sehnengleitfläche nähern, wodurch diese dünn wird und es zu Mikrofrakturen der palmaren Kompakta kommen kann. Primär entsteht somit eine Osteoklasie des subchondralen Knochens.

Beim Auftreten dieser Krankheitssymptome kommt es zu einer wechselhaften chronischen Lahmheit, welche meist zur dauerhaften Unbrauchbarkeit des Pferdes führt.

### STAND DER TECHNIK

Da es sich bei der Podotrochlose um eine der Hauptlahmheitsursachen bei Pferden handelt und angesichts der Häufigkeit der oben beschriebenen Symptomatik, wurden in der Vergangenheit zahlreiche Therapieversuche unternommen, sowohl im pharmazeutischen als auch im chirurgischen und naturheilpraktischen Bereich.

Die EP 0 854 724 B1 beschreibt die Verwendung bestimmter Bisphosphonate, einschließlich Clodronsäure, zur Behandlung der Strahltieinerkrankung bei Pferden. Hinsichtlich der Krankheitsursache werden zwei Theorien in den Vordergrund gestellt, nämlich einerseits eine schlechte Blutzirkulation innerhalb des Beins und andererseits Änderungen in den biomechanischen Eigenschaften des betroffenen Pferdebeins. Die Verabreichung des Arzneimittels kann hierbei enteral, parenteral oder transdermal, vorzugsweise intravenös erfolgen.

Die bisher bekannten Therapieversuche sind jedoch ohne nennenswerten Erfolg verlaufen. Die Konsequenz für das Tier ist ein permanenter Schmerz, verbunden mit einer Bewegungseinschränkung und dem Ausfall des Pferdes für Freizeit oder den Hochleistungssport, und verbunden mit einem erheblichen emotionalen und Kapitalverlust für den Pferdebesitzer.

Kürzlich wurde bekannt, dass eine andere Substanz aus der Klasse der Bisphosphonate, das Tiludronat, einen heilenden Effekt bei Podotrochlose zeigt. Dieser Effekt ist allerdings in einer Vielzahl der Fälle von erheblichen Nebenwirkungen, wie Koliken, begleitet und ferner ist die vorgeschriebene Verabreichung des Wirkstoffs durch Infusion oder intravenöse Injektion für das Tier und den behandelnden Veterinär mit erheblichen Nachteilen verbunden.

### AUFGABE DER ERFINDUNG

Der Erfindung liegt daher die Aufgabe zugrunde, ein wirksames Arzneimittel zur Behandlung von Podotrochlose mit osteoporotischer Beteiligung bei Pferden vorzusehen.

### ZUSAMMENFASSUNG DER ERFINDUNG

Gemäß der Erfindung hat sich nunmehr gezeigt, dass Clodronsäure in wirksamer Weise zur Behandlung von Podotrochlose bei Pferden eingesetzt werden kann, vorausgesetzt, das Arzneimittel wird nach dem in Anspruch 1 definierten Behandlungsschema verabreicht. Insbesondere gelingt es gemäß der Erfindung, osteoporotische Zustände im Bereich des Strahlbeins bei Pferden erfolgreich zu therapieren.

Gegenstand der Erfindung ist somit die Verwendung von Clodronsäure, deren Additionssalze oder Ester, Hydrate oder Salze von Hydraten als Wirkstoff zur Herstellung eines Arzneimittels in Form einer injizierbaren Lösung oder Suspension zur Behandlung von Podotrochlose mit osteoporotischer Beteiligung bei Pferden, wobei die Behandlung durch zwei- bis fünfmalige intramuskuläre Injektion des Arzneimittels innerhalb eines Zeitraums von bis zu mehreren Tagen erfolgt.

Bevorzugte bzw. besonders zweckmäßige Ausgestaltungen des Erfindungsgegenstandes sind in den Unteransprüchen angegeben.

### DETAILLIERTE BESCHREIBUNG DER EREINDUNG

Clodronsäure ist der internationale Freiname für (Dichlormethylen)bis(phosphonsäure). Das Dinatriumsalz der Clodronsäure wird in der Humanmedizin bei Erkrankungen mit Hypercalciämie eingesetzt, insbesondere bei Hypercalciämie in Folge von Knochenmetastasen solider Tumoren oder durch maligne Tumoren induzierte Knochenzerstörungen ohne Knochenmetastasen. Ein Clodronsäure, Dinatriumsalz 4H₂O. enthaltendes Arzneimittel ist beispielsweise unter der Bezeichnung Bonefos^{®} im Handel.

Es hat sich nunmehr gemäß der Erfindung gezeigt, dass mit Clodronsäure außergewöhnliche Heilerfolge bei der Behandlung von Podotrochlose mit osteoporotischer Beteiligung bei Pferden erzielt werden können, sodass der größte Teil der behandelten Pferde wieder einer regelmäßigen Nutzung als Reit- bzw. Sportpferd zugeführt werden kann.

Gemäß der Erfindung eignen sich als Additionssalze der Clodronsäure insbesondere die Alkalimetallsalze, wobei das Dinatriumsalz, insbesondere das Dinatriumsalz 4H₂O, am meisten geeignet und bevorzugt ist.

Das erfindungsgemäß eingesetzte Arzneimittel in Form einer injizierbaren Lösung oder Suspension kann übliche Hilfs- und Trägersubstanzen enthalten, wie etwa Bindemittel, Puffersubstanzen, Gleitmittel, Verdünnungsmittel und Farbstoffe. Die Herstellung der injizierbaren Lösung oder Suspension erfolgt in an sich bekannter Weise.

Um den erwünschten Heilerfolg, auch prophylaktisch, zu erzielen, ist die Einhaltung eines Verabreichungsregimes notwendig, bei dem das Arzneimittel durch zwei- bis fünfmalige intramuskuläre Injektion innerhalb eines Zeitraums von bis zu mehreren Tagen, wie etwa 3 Folgetagen, vorzugsweise jedoch innerhalb eines Tages, verabreicht wird. Hierbei kann die Verabreichung des Arzneimittels in einer Dosierung je Injektion von 0, 125-2,5 mg, vorzugsweise 0.25-1 mg, insbesondere bevorzugt etwa 0.6 mg Wirkstoff/kg Körpergewicht erfolgen. Bei einem Pferd mit einem Gewicht von 500 kg wird eine Gesamtdosis von 250-2500 mg, vorzugsweise 400-1500 mg, insbesondere bevorzugt etwa 800 bis 1000 mg Wirkstoff, verteilt auf 2 bis 5 Injektionen, vorzugsweise 3 Injektionen, angestrebt. Bei einem Pferd mit einem Gewicht von 500 kg werden somit besonders vorzugsweise 900 mg Wirkstoff, verteilt auf 3 Injektionen von je 300 mg, innerhalb eines Tages verabreicht. Die intramuskuläre Injektion erfolgt vorzugsweise in die Hals-, Brust- und Glutaeus-Muskulatur des Pferdes. Bei 3 Injektionen werden vorzugsweise zwei Injektionen in die Halsmuskulatur (linke und rechte Seite), insbesondere bevorzugt etwa eine Handbreit unter dem Mähnenkamm und etwa eine Handbreit vor der Scapula, und eine Injektion in die Brustmuskulatur verabreicht. In zweckmäßiger Weise erfolgen die 2 bis 5 Injektionen in direkter Aufeinanderfolge.

Nach etwa 14 Tagen tritt hierbei bei fast allen Pferden eine deutliche Verbesserung der klinischen Symptomatik ein. Nach etwa 4-6 Wochen hat sich das klinische Bild soweit stabilisiert und eingestellt, dass man den Heilerfolg definieren kann. Nach Ablauf von etwa 10 bis 14 Monaten verschlechtert sich in der Regel die klinische Symptomatik. Dann kann die Behandlung mit dem gleichen Erfolg ein- oder mehrmals wiederholt werden. Auf diese Weise gelingt es, die behandelten Pferde mehrere Jahre lahmheitsfrei zu halten.

Bisher sind mindestens 350 Pferde, welche an Podotrochlose leiden und mit herkömmlichen Therapien ohne Erfolg vorbehandelt waren, erfolgreich und nebenwirkungsfrei mit dem erfindungsgemäß beschriebenen Arzneimittel therapiert worden. Viele der so behandelten Pferde sind unter regelmäßigem Einsatz dieses Arzneimittels seit mehreren Jahren lahmheitsfrei. Beispielsweise war der Therapieerfolg bei einem Wallach, der an Podotrochlose litt und ca. 3 Jahre nicht geritten oder gar im Sport eingesetzt werden konnte, nach dem Einsatz des erfindungsgemäß beschriebenen Arzneimittels so gut, dass der Wallach danach noch 6 Jahre intensiv und erfolgreich im Springsport eingesetzt werden konnte.

## Patentansprüche

1. Clodronsäure, deren Additionssalze oder Ester, Hydrate oder Salze von Hydraten zur Verwendung als Arzneimittel in Form einer injizierbaren Lösung oder Suspension zur Behandlung von Podotrochlose mit osteoporotischer Beteiligung bei Pferden, wobei die Behandlung durch zwei- bis fünfmalige intramuskuläre Injektion des Arzneimittels innerhalb eines Zeitraums von bis zu etwa 3 Folgetagen erfolgt.

2. Clodronsäure, deren Additionssalze oder Ester, Hydrate oder Salze von Hydraten, zur Verwendung nach Anspruch 1, wobei als Additionssalze Alkalimetallsalze eingesetzt werden.

3. Clodronsäure, deren Additionssalze oder Ester, Hydrate oder Salze von Hydraten, zur Verwendung nach Anspruch 1 und/oder 2, wobei das Dinatriumsalz der Clodronsäure eingesetzt wird.

4. Clodronsäure, deren Additionssalze oder Ester, Hydrate oder Salze von Hydraten, zur Verwendung nach mindestens einem der Ansprüche 1-3, wobei das Arzneimittel übliche Hilfs- und Trägersubstanzen enthält.

5. Clodronsäure, deren Additionssalze oder Ester, Hydrate oder Salze von Hydraten, zur Verwendung nach mindestens einem der Ansprüche 1-4, wobei die Behandlung durch zweibis fünfmalige intramuskuläre Injektion des Arzneimittels innerhalb eines Tages erfolgt.

6. Clodronsäure, deren Additionssalze oder Ester, Hydrate oder Salze von Hydraten, zur Verwendung nach mindestens einem der Ansprüche 1-5, wobei die Behandlung nach Ablauf von 10-14 Monaten ein- oder mehrmals wiederholt wird.

7. Clodronsäure, deren Additionssalze oder Ester, Hydrate oder Salze von Hydraten, zur Verwendung nach mindestens einem der Ansprüche 1-6, wobei das Arzneimittel in einer Dosierung je Injektion von 0,125-2,5 mg, vorzugsweise 0,25-1 mg, insbesondere bevorzugt etwa 0,6 mg Wirkstoff/kg Körpergewicht verabreicht wird.

## Claims

1. Clodronic acid, its addition salts or esters, hydrates or salts of hydrates for use as a pharmaceutical in the form of an injectable solution or suspension for the treatment of podotrochlosis with associated osteoporosis in horses, the treatment being carried out by two to five intramuscular injections of the pharmaceutical within a period of up to approximately 3 consecutive days.

2. Clodronic acid, its addition salts or esters, hydrates or salts of hydrates for use according to claim 1, wherein the addition salts employed are alkali metal salts.

3. Clodronic acid, its addition salts or esters, hydrates or salts of hydrates for use according to claim 1 and/or 2, wherein the disodium salt of clodronic acid is employed.

4. Clodronic acid, its addition salts or esters, hydrates or salts of hydrates for use according to claims 1-3, wherein the pharmaceutical comprises customary adjuvants and excipients.

5. Clodronic acid, its addition salts or esters, hydrates or salts of hydrates for use according to claims 1-4, wherein the treatment is carried out by two to five intramuscular injections of the pharmaceutical within one day.

6. Clodronic acid, its addition salts or esters, hydrates or salts of hydrates for use according to claims 1-5, wherein the treatment is repeated once or more than once after 10-14 months have elapsed.

7. Clodronic acid, its addition salts or esters, hydrates or salts of hydrates for use according to claims 1-6, wherein the pharmaceutical is administered at a dosage, per injection, of 0.125-2.5 mg, preferably 0.25-1 mg, particularly preferably approximately 0.6 mg of active ingredient/kg body weight.

## Revendications

1. Acide clodronique, ses sels d'addition ou ses esters, hydrates ou sels d'hydrates, destinés à une utilisation comme médicament sous forme d'une solution ou suspension injectable permettant de traiter le syndrome podotrochléaire associé à des symptômes d'ostéoporose chez le cheval, ledit traitement consistant en deux à cinq injections intramusculaires dudit médicament, réalisées dans un intervalle pouvant comprendre jusqu'à environ 3 jours consécutifs.

2. Acide clodronique, ses sels d'addition ou ses esters, hydrates ou sels d'hydrates, destinés à une utilisation selon la revendication 1, les sels d'addition mis en oeuvre à cet effet étant des sels de métaux alcalins.

3. Acide clodronique, ses sels d'addition ou ses esters, hydrates ou sels d'hydrates, destinés à une utilisation selon les revendications 1 et/ou 2, ledit acide clodronique étant mis en oeuvre sous forme de son sel de disodium.

4. Acide clodronique, ses sels d'addition ou ses esters, hydrates ou sels d'hydrates, destinés à une utilisation selon au moins une des revendications 1 à 3, ledit médicament contenant des excipients et véhicules usuels.

5. Acide clodronique, ses sels d'addition ou ses esters, hydrates ou sels d'hydrates, destinés à une utilisation selon au moins une des revendications 1 à 4, ledit traitement consistant en deux à cinq injections intramusculaires dudit médicament, réalisées dans la même journée.

6. Acide clodronique, ses sels d'addition ou ses esters, hydrates ou sels d'hydrates, destinés à une utilisation selon au moins une des revendications 1 à 5, ledit traitement étant répété une ou plusieurs fois au terme de 10 à 14 mois.

7. Acide clodronique, ses sels d'addition ou ses esters, hydrates ou sels d'hydrates, destinés à une utilisation selon au moins une des revendications 1 à 6, ledit médicament étant administré selon une posologie consistant à mettre en oeuvre, par injection, 0,125 à 2,5 mg, préférentiellement 0,25 à 1 mg, de préférence notamment 0,6 mg de principe actif / kg de poids corporel.
